# EUROPEAN PATENT APPLICATION

(11) **EP 4 588 508 A1**
(43) Date of publication of application: **23.07.2025**
(21) Application number: 23902463.1
(22) Date of filing: 24.11.2023
(51) Int. Cl.: A61N 5/06

(54) **BEAUTIFICATION INSTRUMENT**

(30) Priority: 16.12.2022 CN 202223396826 U; 16.12.2022 CN 202223427528 U
(71) Applicant: One Beauty Technology Co., Ltd, Shenzhen, Guangdong 518000 (CN)
(72) Inventor: PAN, Ninghua, Shenzhen, Guangdong 518000 (CN)
(74) Representative: Studio Torta S.p.A.
(86) International application number: PCT/CN2023/133947
(87) International publication number: WO 2024/125265

(57) **Abstract**

Disclosed are a beautification instrument, comprising a base unit and a lamp head. The base unit comprises a first housing and a control motherboard disposed inside the first housing. The lamp head can be electrically connected to the control mainboard, and the control mainboard is configured to provide a control signal and a working current for the lamp head. The lamp head comprises a second housing, and a pulse light source assembly and a phototherapy assembly disposed inside the second housing. The second housing is separated from the first housing, and a light-emitting channel is disposed in the second housing. The pulse light source assembly is disposed opposite to the light-emitting channel, and the pulse light emitted by the pulse light source assembly can be emitted from a front end of the light-emitting channel. The light-emitting direction of the phototherapy assembly is the same as the light-emitting direction of the pulse light source assembly.

## Description

### CROSS-REFERENCE TO RELATED APPLICATIONS

This application claims priority to a Chinese patent application No. 202223396826.3, titled "Beautification Instrument", filed on December 16, 2022, and a Chinese patent application No. 202223427528.6, titled "Beautification Instrument System and Beautification Instrument Thereof", filed on December 16, 2022, all of which are incorporated into this application by reference.

### TECHNICAL FIELD

The present application relates to the technical field of a beautification instrument, particularly to a beautification instrument.

### BACKGROUND

As a device having the functions of removing spots, reducing or eliminating wrinkles, improving skin condition, removing the body hair from the surface of the skin, etc., the beautification instrument is becoming more and more popular, and people's requirements for it are also becoming higher and higher. The existing beautification instrument is mainly composed of a lamp head and a body. The lamp head generally includes a pulse light source, which directly irradiates the skin through the strong pulse light emitted by the pulse light source, causing the skin to produce a photobiochemical effect, to achieve the corresponding cosmetic effect. In order to be able to achieve different cosmetic effects, the existing lamp head will also be provided with other optical assemblies. The body provides power and control signals for the lamp head.

The existing beautification instrument is generally formed integratedly by the lamp head and body, with a larger volume, occupying more space, and not conducive to being stored, etc.

Thus, the existing technology needs to be improved and enhanced.

### SUMMARY

The present application provides a beautification instrument in which the lamp head can be separated from the body, to improve the user's experience of using the device.

An embodiment of the present application provides a beautification instrument, including:
a body, comprising a first housing and a control main board provided inside the first housing;
a lamp head, electrically connected to the control main board, the control main board is configured to to provide control signals and working current for the lamp head, and the lamp head includes a second housing, and a pulse light source assembly and a light therapy assembly provided within the second housing;
the second housing is provided separately from the first housing, and a light outlet channel is provided inside the second housing, the pulse light source assembly is opposite to the light outlet channel, and pulse light emitted by the pulse light source assembly is ejectable from a front end of the light outlet channel, and light is emitted by the light therapy assembly in a same direction as light emitted by the pulse light source assembly.

In one embodiment, the beautification instrument includes a connection assembly, the body and the lamp head are connected via the connection assembly.

In one embodiment, the lamp head is provided separately from the body; and
the connection assembly is a connection cable, an end of the connection cable is physically and electrically connected to the body, and another end of the connection cable is physically and electrically connected to the lamp head.

In one embodiment, the connection cable is detachably connected to at least one of the body and the lamp head;
an end of the connection cable is electrically connected to the lamp head and another end of the connection cable is electrically connected to the body, and a control main board of the body is electrically connected to the lamp head via the connection cable.

In one embodiment, two ends of the connection cable are plugged with the body and the lamp head, respectively;
the connection cable includes an elastic portion, a first connector and a second connector provided at two ends of the elastic portion, the body is provided with a first interface, the lamp head is provided with a second interface, the first connector is pluggable into the first interface, and the second connector is pluggable into the second interface.

In one embodiment, an end of the connection cable is fixed to the body, and an electrical connection of the connection cable and the body is kept, and another end of the connection cable is fixed to the lamp head, and an electrical connection of the connection cable and the lamp head is kept.

In one embodiment, the body is snapped to the lamp head by the connection assembly;
the connection assembly is a snap-fit and a snapping seat for cooperation, the snap-fit is provided at a front end of the body and the snapping seat is provided at a rear end of the lamp head; or
the snap-fit is provided at a rear end of the lamp head and the snapping seat is provided at a front end of the body.

In one embodiment, the snap-fit is protruded at the rear end of the second housing, and the snap-fit includes a convex extension and a snapping portion, an angle is formed between the snapping portion and the convex extension, and the convex extension is connected to a rear end of the second housing; and
the snap-fit is provided at the front end of the first housing, the snap-fit is provided with a snapping groove with an opening forward, and the snapping portion is snapped in the snapping groove when the lamp head is docked with the body.

In one embodiment, the connection assembly further includes a limitation member, the limitation member is provided in one of the body and the lamp head, and another one of the body and the lamp head is provided with a limitation groove corresponding to the limitation member, the limitation member is movably accessed into the limitation groove to lock the lamp head and the body.

In one embodiment, the limitation member is provided at the front end of the first housing, the limitation member includes a pressing portion and a limitation portion, the pressing portion is protruded from one of sides of the first housing, and the limitation portion is protruded from the front end of the first housing; and
the limitation groove is provided at a rear end of the second housing, and the limitation portion is movably accessed into the limitation groove.

In one embodiment, plug-in connection terminal sets adapted to be connected to each other are provided at surfaces where the body is docked with the lamp head.

In one embodiment, the plug-in connection terminal sets comprises a first connection terminal and a second connection terminal, the first connection terminal is provided at the front end of the body, the second connection terminal is provided at the rear end of the lamp head, and the first connection terminal is pluggable into the second connection terminal.

In one embodiment, the connection assembly further includes a first insertion groove provided at the front end of the body and a second insertion groove provided at the rear end of the lamp head, the first connection terminal is provided in the first insertion groove, the second connection terminal is provided in the second insertion groove, and the first connection terminal is pluaggable with the second connection terminal when the first insertion groove is pluagged in the second insertion groove.

In one embodiment, the light therapy assembly is provided at a front end of the second housing;
the light therapy assembly comprises a light plate with a plurality of light emitting diode (LED) light beads, the light plate is provided with through holes in communication with the light outlet channel; and
the plurality of the LED light beads are electrically connected to the light plate, and provided on the light plate along a circumferential direction of the light plate, and the plurality of light outlets are provided on a front end of the second housing, and each of the plurality of the light outlets is provided with one LED light bead.

In one embodiment, the light therapy assembly is provided at a rear end inside the light outlet channel;
the light therapy assembly includes a light plate and the plurality of the LED light beads, and the light plate is provided with through holes in communication with the light outlet channel; and
the plurality of the LED light beads are electrically connected to the light plate, provided on the light plate along the circumferential direction of the light plate, and towards an outlet end at the front end of the light outlet channel.

In one embodiment, a temperature sense element is provided at the front end of the second housing, a mounting hole is provided at the front end of the second housing, the temperature sense element is accommodated in the mounting hole and passed through the mounting hole, and is configured to detect a temperature signal from a skin.

In one embodiment, a plurality of the temperature sense elements are provided at the front end of the second housing in a circumferential direction, each of a plurality of mounting holes is provided with one temperature sense element, and the plurality of the temperature sense elements are passed through the plurality of the the mounting holes one-in-one.

In one embodiment, the lamp head further includes a heat conductive plate provided on the front end of the second housing, and the heat conductive plate is provided in a front end of the temperature sense element, the temperature sense element is in contact with the heat conductive plate; and
the heat conductive plate is provided with an opening hole in communication with the light outlet channel.

In one embodiment, the lamp head further includes a spotlight frame provided in the second housing, the spotlight frame is extended in a length direction of the beautification instrument, and has openings at front and rear ends of the spotlight frame to form the light outlet channel, and the spotlight frame has an inner diameter that gradually increases from front to back;
the lamp head further includes a support frame provided around an outer periphery of the spotlight frame.

In one embodiment, the pulse light source assembly includes a light source, a reflector, and an optical filter, wherein the light source is fixed on an inner side of the reflector, and the light source is a xenon gas tube;
the second housing is also provided with a mounting frame, the mounting frame is located at a rear end of the reflector and fixed to the reflector; and
the optical filter is located at a front end of the reflector and at a rear end of the light outlet channel.

Compared to the related art, in the beautification instrument provided in the present application, since the first housing of the body is provided separately from the second housing of the lamp head, the body can be separated from the lamp head. The body and the lamp head occupy less space respectively, to be stored easily. Moreover, the lamp head is provided with a light therapy assembly, and light is emitted by the light therapy assembly in a same direction as light emitted by the pulse light source assembly, so that while the pulse light source assembly emits pulse light to remove hair, spot, etc., the light therapy assembly can repair the skin, reduce the stinging sensation brought about by damaging the skin, and improve the user's sense of experience.

### BRIEF DESCRIPTION OF THE DRAWINGS

FIG. 1 is a three-dimensional structure view of the beautification instrument in this embodiment.
FIG. 2 is a decomposed structure view of the beautification instrument in this embodiment.
FIG. 3 is a three-dimensional structure view of the beautification instrument in other embodiments.
FIG. 4 is a three-dimensional structure view of the body in other embodiments.
FIG. 5 is a three-dimensional structure view of a lamp head in other embodiments.
FIG. 6 is a sectional view of the lamp head in this embodiment.
FIG. 7 is a three-dimensional structure view of the lamp head in another embodiment.
FIG. 8 is a partial structure view of the lamp head in another embodiment.
FIG. 9 is a sectional view of the lamp head in another embodiment.

1, a body; 11, a first housing; 12, a first interface; 13, a power input terminal;
2, a lamp head; 21, a second housing; 22, a second interface; 23, a spotlight frame; 241, a light outlet channel; 242, a light outlet; 25, a support frame; 261, a light source; 262, a reflector; 263, an optical filter; 27, a mounting frame; 28, a light therapy assembly; 281, a lamp plate; 2811, a through hole; 282, an LED lamp bead;
31, a connection cable; 311, an elastic portion; 312, a first connector; 313, a second connector;
41, a snap-fit; 411, a convex extension; 412, a snapping portion; 42, a snapping seat; 421, a snapping groove; 43, a limitation member; 431, a pressing portion; 432, a limitation portion; 44, a limitation groove; 451, a first insertion groove; 452, a second insertion groove;
51, a first connection terminal; 52, a second connection terminal;
6, a temperature sense element;
7, a heat conductive plate.

### DETAILED DESCRIPTION OF THE EMBODIMENTS

The present application provides a beautification instrument, and in order to make the purpose, technical solution and effect of the present application clearer and more explicit, the present application is described in further detail hereinafter with reference to the accompanying drawings and by way of embodiments. It should be understood that the specific embodiments described herein are only for explaining the present application and are not intended to limit the present application.

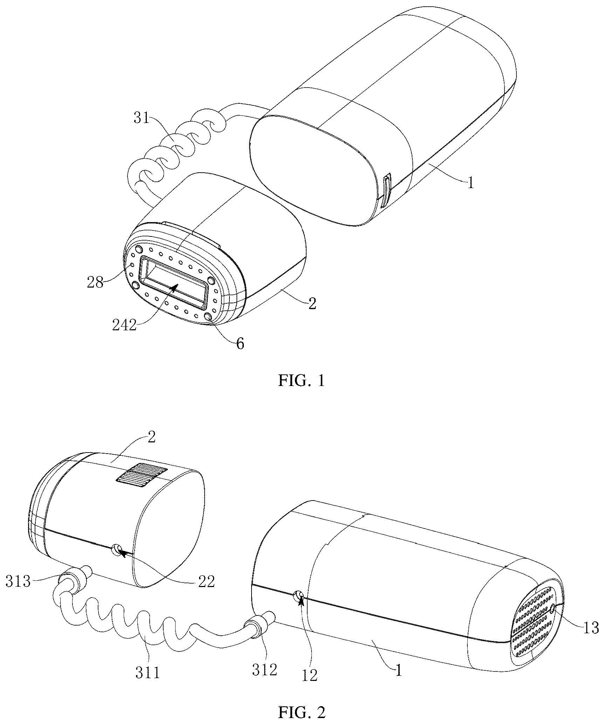

Referring to FIGS. 1 and 2, FIG. 1 is a three-dimensional structure view of the beautification instrument in the present embodiment, and FIG. 2 is a decomposed structure view of the beautification instrument in the present embodiment.

The beautification instrument in this embodiment includes a body 1 and a lamp head 2. The body 1 includes a first housing 11 and the lamp head 2 includes a second housing 21, and the first housing 11 of the body 1 and the second housing 21 of the lamp head 2 are separated, so that the body 1 and the lamp head 2 can be separated. Compared to the related art in which the lamp head 2 and the body 1 are integrally formed, the body 1 and the lamp head 2 in the present embodiment can be separated from each other, so that the body 1 and the lamp head 2 respectively occupy less space, which is convenient for storage, and for replacement of the lamp head 2 or the body 1.

For ease of description, it is hereby provided that along the length of the beautification instrument, an end of of the beautification instrument facing the lamp head 2 is a front end and the end of of the beautification instrument facing the body 1 is a rear end.

The body 1 includes a first housing 11 and a control main board provided inside the first housing 11. In this embodiment, the control main board is a printed circuit board.

The body 1 is also provided with a battery inside. The first housing 11 is provided with an accommodation cavity, the battery is provided in the accommodation cavity, and the battery is electrically connected to the control main board to power the entire beautification instrument. Specifically, the battery may be a lithium battery.

Further, a rear end of the body 1 is provided with a power input terminal 13, the power input terminal 13 is electrically connected to the battery, and the control main board controls the power to be input. Since the battery is electrically connected to the control main board, the control main board can control charging the battery when the power input terminal 13 is connected to the power source.

In other embodiments, there may be a plurality of bodies 1, and each body 1 is provided with a battery. When the power of the battery in one body 1 is consumed, another body 1 with a fully charged battery can be replaced immediately, so that the plurality of bodies 1 can be used alternately, thereby increasing the continuous use time of the beautification instrument.

The lamp head 2 is provided separately from the body 1, so that the body 1 and the lamp head 2 occupy less space respectively, to be stored easily.

In other embodiments, there maybe a plurality of lamp heads 2, and the power of each lamp head 2 is different, and the shape of the light outlet 242 is different, so that it is convenient for the user to select the appropriate lamp head 2 according to his or her own use needs. Therefore, the beautification instrument can be used in multiple ways to meet various use needs. It avoids the user from repeatedly purchasing the beautification instrument with different functions for many times, resulting in a waste of resources.

The beautification instrument includes a connection assembly, and the body 1 and the lamp head 2 are connected by the connection assembly.

In this embodiment, the connection assembly is a connection cable 31, and the body 1 and the lamp head 2 are physically and electrically connected via the connection cable 31. The connection cable 31 has a certain length, so that the lamp head 2 and the body 1 can be used separately. On the one hand, the weight of the lamp head 2 is small, and when using the beautification instrument, only the lamp head 2 needs to be held, which can reduce the weight. On the other hand, the volume of the lamp head 2 is small, it can be operated flexibly, thereby improving the user's sense of use experience. Specifically, an end of the connection cable 31 is physically and electrically connected to the body 1, and another end of the connection cable 31 is physically and electrically connected to the lamp head 2.

The connection cable 31 is detachably connected to at least one of the body 1 and the lamp head 2, and an end of the connection cable 31 is connected to the lamp head 2 and another end of the connection cable 31 is electrically connected to the body 1. At this time, the control main board in the body 1 of the connection cable 31 is electrically connected to the lamp head 2 via the connection cable 31.

In this embodiment, two ends of the connection cable 31 are detachably connected to the body 1 and the lamp head 2, respectively, to overhaul or replace the lamp head 2 or the body 1, conveniently.

Specifically, two ends of the connection cable 31 are plugged with the body 1 and the lamp head 2, respectively. The connection cable 31 includes an elastic portion 311 and a first connector 312 and a second connector 313 provided at two ends of the elastic portion 311. The body 1 is provided with a first interface 12, and the lamp head 2 is provided with a second interface 22, and the first connector 312 can be inserted into the first interface 12, and the second connector 313 can be inserted into the second interface 22, and the lamp head 2 is electrically connected to the body 1.

Moreover, the elastic portion 311 is in the shape of a spiral, which is elastic and can be stretched when subjected to an external force, so that when the beautification instrument is used, it will not be limited by the length of the connection cable 31, and is easy to be used, and then, it can be naturally retracted to be stowed easily when the external force is removed.

In some embodiments, one of the ends of the connection cable 31 is detachably connected to the body 1 and another end of the connection cable 31 is fixed to the lamp head 2. Alternatively, one of the ends of the connection cable 31 is detachably connected to the lamp head 2 and another end of the connection cable 31 is fixed to the body 1.

In other embodiments, one of ends of the connection cable 31 is fixed to the body 1, and an electrical connection of the connection cable 31 and the body 1 is kept, and another end of the connection cable 31 is fixed to the lamp head 2, and an electrical connection of the connection cable 31 and the lamp head 2 is kept.

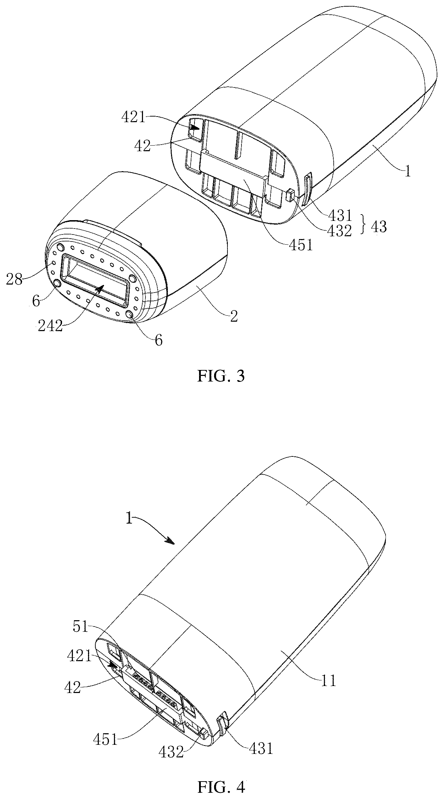

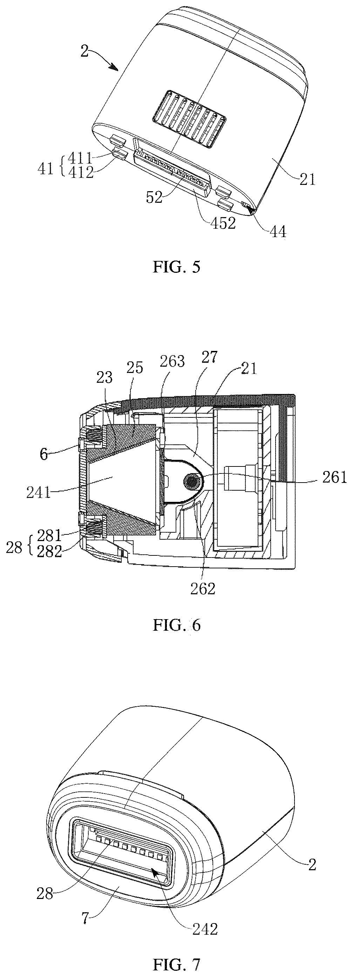

Referring to FIGS. 3 to 5, FIG. 3 is a three-dimensional structure view of the beautification instrument in other embodiments, FIG. 4 is a three-dimensional structure view of the body 1 in other embodiments, and FIG. 5 is a three-dimensional structure view of the lamp head 2 in other embodiments. The body 1 is snapped to the lamp head 2 for fixation by a connection assembly.

The connection assembly includes a snap-fit 41 and a snapping seat 42 for cooperation. The snap-fit 41 is provided at the rear end of the lamp head 2, and the snapping seat 42 is provided at the front end of the body 1. Specifically, the snap-fit 41 is protruded at the rear end of the second housing 21, and the snapping seat 42 is provided at the front end of the first housing 11. The snap-fit 41 includes a convex extension 411 and a snapping portion 412 connected to each other, an angle is formed between the snapping portion 412 and the convex extension 411, and the convex extension 411 is connected to the rear end of the second housing 21. The angle may be an acute angle or a right angle, so that the snap-fit 41 is L-shaped. The shape of the snapping groove 421 corresponds to that of the snap-fit 41, so that when the lamp head 2 is docked with the body 1, the snapping portion 412 is snapped in the snapping groove 421 to realize the snap-fit fastening of the lamp head 2 with the body 1.

In some embodiments, the snap-fit 41 is provided at the front end of the body 1 and the snapping seat 42 is provided at the rear end of the lamp head 2.

In this embodiment, four snaps 41 are distributed in a matrix at the rear end of the lamp head 2. Similarly, four snaps 421 are distributed in a matrix at the front end of the body 1.

Referencing to FIGS. 3 to 5, the connection assembly further includes a limitation member 43, the limitation member 43 is provided in one of the body 1 and the lamp head 2, and the other one of the body 1 and the lamp head 2 is provided with a limitation groove 44 that corresponds to the limitation member 43, and the limitation member 43 can be movably accessed into the limitation groove 44, to lock the lamp head 2 and the body 1.

Specifically, the limitation member 43 is provided at the front end of the first housing 11, and the limitation groove 44 is provided at the rear end of the second housing 21. The limitation member 43 includes a pressing portion 431 and a limitation portion 432, the pressing portion 431 is protruded from one of the sides of the first housing 11, and the limitation portion 432 is protruded from the front end of the first housing 11. The limitation portion 432 can be movably accessed in the limitation groove 44, to lock the lamp head 2 and the body 1.

Referencing to FIGS. 3 to 5, in this embodiment, when the lamp head 2 is connected to the body 1, the lamp head 2 can be electrically connected to a control main board within the first housing 11 of the body 1. Specifically, plug-in connection terminal sets adapted to be electrically connected to each other are provided at surfaces where the body 1 is docked with the lamp head 2, and the lamp head 2 is electrically connected to the control main board in the body 1 through the plug-in connection terminal sets, and at this time, the control main board can provide control signals and working currents for the lamp head 2, to realize the work of the lamp head 2. The connection terminal sets includes a first connection terminal 51 and a second connection terminal 52, and the first connection terminal 51 is provided at the front end of the body 1 and the second connection terminal 52 is provided at the rear end of the lamp head 2. The first connection terminal 51 is pluggable with the second connection terminal 52, and an electrical connection of the lamp head 2 and the body 1 is kept after plugging in.

In some embodiments, the first connection terminal 51 may also be provided at the rear end of the lamp head 2 and the second connection terminal 52 corresponds to the front end of the body 1.

In this embodiment, the connection assembly further includes a first insertion groove 451 provided at the front end of the body 1 and a second insertion groove 452 provided at the rear end of the lamp head 2, and the first insertion groove 451 and the second insertion groove 452 have openings opposite to each other. The first connection terminal 51 is provided in the first insertion groove 451 and the second connection terminal 52 is provided in the second insertion groove 452 to protect the first connection terminal 51 and the second connection terminal 52. When the lamp head 2 is docked with the body 1, the first insertion groove 451 is docked with the second insertion groove 452, so that the first connection terminal 51 is plugged in the second connection terminal 52, to realize the electrical connection of the lamp head 2 and the control main board in the body 1.

Referring to FIGS. 5 and 6, FIG. 6 is an exploded view of the lamp head 2 in this embodiment. The lamp head 2 includes a second housing 21 and a pulse light source assembly and a light therapy assembly 28 provided inside the second housing 21.

A volume of the second housing 21 is smaller than the volume of the first housing 11. The second housing 21 is configured to house the pulse light source assembly and the light therapy assembly 28. The second housing 21 is provided separately from the first housing 11.

The second housing 21 is provided with a light outlet channel 241, and a light outlet 242 is formed at a front end of the second housing 21, and the light outlet 242 is in communication with the light outlet channel 241. In this embodiment, the light outlet 242 has a square shape. In some embodiments, the shape of the light outlet 242 may also be circular or other shapes.

Specifically, the lamp head 2 further includes a spotlight frame 23. The spotlight frame 23 is provided within the second housing 21. The spotlight frame 23 extends along the length direction of the beautification instrument and has openings at the front and rear ends to form the light outlet channel 241. Moreover, the inner diameter of the spotlight frame 23 gradually increases from front to back to collect the light.

In this embodiment, the spotlight frame 23 may have a square cross-section along the width direction of the beautification instrument. In other embodiments, the cross-section of the spotlight frame 23 along the width direction of the beautification instrument may be round, which is specifically determined as needed.

Further, the lamp head 2 further includes a support frame 25, which is provided around the periphery of the spotlight frame 23, and configured to to support the spotlight frame 23.

The pulse light source assembly is configured to emit pulse light. The pulse light source assembly is provided inside the second housing 21 at the rear end of the light outlet channel 241, i.e., the pulse light source assembly is provided at the rear end of the spotlight frame 23, and the pulse light source assembly is opposite to the light outlet channel 241. The light emitted from the pulse light source assembly is in a direction toward the front end of the lamp head 2, i.e., the pulse light emitted by the pulse light source assembly can be emitted from the front end of the light outlet channel 241.

The pulse light source assembly includes a light source 261, a reflector 262, and a optical filter 263, and the light source 261 is fixed on the inner side of the reflector 262. In this embodiment, the light source 261 is a xenon lamp.

The second housing 21 is also provided with a mounting frame 27, which is located at the rear end of the reflector 262, and the mounting frame 27 is fixed to the reflector 262.

An optical filter 263 is provided at the front end of the reflector 262 and is located at the rear end of the light outlet channel 241 for filtering harmful ultraviolet light and light whose wavelength is too short and has insufficient penetrating power.

The light therapy assembly 28 is provided on the second housing 21, and the light emitted from the light therapy assembly 28 is in the same direction as that in the pulse light source assembly, i.e., the light emitted from the light therapy assembly 28 and the pulse light source assembly are both directed toward the front end of the lamp head 2, for repairing the skin while the light emitted from the pulse light source assembly is used to remove hair.

In this embodiment, the light therapy assembly 28 is provided at the front end of the second housing 21. The light therapy assembly 28 includes a light plate 281 and a plurality of LED light beads 282. The light plate 281 is provided with a through hole 2811 in communication with the light outlet channel 241 to expose the light outlet 242 of the light outlet channel 241 for the light emitted by the pulse light source assembly passing through and ejecting conveniently. Specifically, the light plate 281 is in the shape of a mouth. A plurality of LED light beads 282 are electrically connected to the light plate 281, and provided on the light plate 281 along a circumferential direction.

In order to transmit the light emitted by each LED light bead 282, a plurality of light outlets are provided on a front surface of the second housing 21. The plurality of light outlets surround the periphery of the outlet end of the light outlet channel 241, i.e., the plurality of light outlets surround the periphery of the light outlet 242 of the light outlet channel 241. Moreover, each of the plurality of the light outlets is provided with an LED light bead 282, so that the LED light bead 282 is closer to the user's skin, thereby improving the repair effect of the light therapy assembly 28.

In this embodiment, the light therapy assembly 28 is a red LED light.

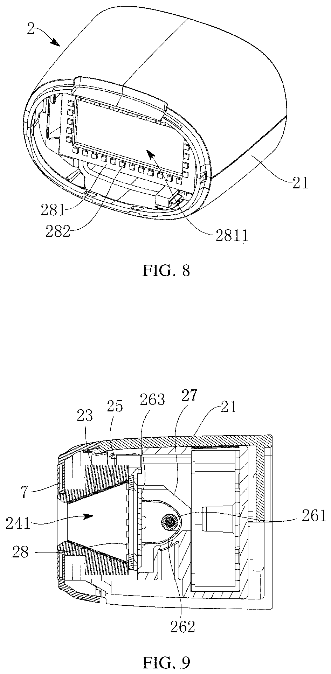

Referring to FIGS. 7 to 9, FIG. 7 is a three-dimensional structure view of the lamp head 2 in another embodiment, FIG. 8 is a local structure view of the lamp head 2 in another embodiment, and FIG. 9 is a sectional view of the lamp head 2 in another embodiment. In this embodiment, the light therapy assembly 28 is provided at a rear end inside the light outlet channel 241. The light therapy assembly 28 includes a lamp plate 281 and a plurality of LED light beads 282. The lamp plate 281 is provided with a through hole 2811 in communication with the light outlet channel 241 to expose a light source 261 of the pulse light source assembly to be opposite to the light outlet channel 241, such that the light emitted from the light source 261 of the pulse light source assembly pass through and is emitted out. Specifically, the light plate 281 is in the shape of a mouth. A plurality of LED light beads 282 are electrically connected to the light plate 281, and provided on the light plate 281 in a circumferential direction, and toward an outlet end at a front end of the light outlet channel 241, i.e., the light outlet surface of the LED light beads 282 faces the light outlet 242.

Referring to FIGS. 1 and 6, in this embodiment, the front end of the second housing 21 is provided with a temperature sense element 6. The front end of the second housing 21 is provided with a mounting hole, the temperature sense element 6 is accommodated in the mounting hole, and the temperature sense element 6 passes through the mounting hole and is protruded forwardly out of the mounting hole, that is, it is protruded forwardly out of the second housing 21, to be in contact with the user's skin for detecting a temperature signal of the user's skin and controlling the beautification instrument to stop working when the detected temperature signal exceeds a preset value, thereby preventing the user's skin from being burned due to an excessively high temperature.

A plurality of the temperature sense elements 6 are provided on the front surface of the second housing 21 in a circumferential direction. There are plurality of mounting holes, and each mounting hole is correspondingly provided with one temperature sense element 6, and the temperature sense elements 6 pass through the mounting holes one-in-one, and protrudes forwardly out of the mounting holes, to facilitate the contact with the user's skin. Specifically, four temperature sense elements 6 are located at four corners of the front end of the second housing 21.

Referring to FIGS. 7 to 9, in this embodiment, there maybe one temperature sense element 6. Specifically, a front end of the second housing 21 is provided with one temperature sense element 6. The front end of the second housing 21 is provided with a mounting hole for accommodating the temperature sense element 6, and the temperature sense element 6 is passed through the mounting hole and protruded forwardly out of the mounting hole. At this time, the temperature sense element 6 is provided adjacent to the front end of the light outlet channel 241, i.e., the temperature sense element 6 is provided at the front end of the second housing 21 and is located at the outer periphery of the light outlet 242.

Further, the lamp head 2 also includes a heat conductive plate 7 provided on the front surface of the second housing 21, and the heat conductive plate 7 is provided at the front end of the temperature sense element 6. The temperature sense element 6 is in contact with the heat conductive plate 7, and can directly contact the user's skin, so as to expand the area where the temperature sense element 6 can collect temperature signals. When the beautification instrument is used, the heat conductive plate 7 is in contact with the user's skin, and the temperature sense element 6 detects the temperature signal of the user's skin by detecting the temperature of the heat conductive plate 7, and controls the beautification instrument to stop working when the temperature signal detected by the temperature sense element 6 exceeds a preset value, thereby preventing the user's skin from being burned. Specifically, the heat conductive plate 7 is a metal plate.

In summary, the beautification instrument in the present application has at least the following beneficial effects:
First, in the beautification instrument provided in the present application, since the first housing of the body and the second housing of the lamp head are separated, the body can be separated from the lamp head, and the space occupied by the body and the lamp head is smaller, to to be stored easily. Moreover, the lamp head is provided with a light therapy assembly, and the light emitted from the light therapy assembly is in the same direction as that of the pulse light source assembly, so that while the pulse light source assembly emits pulse light to remove hair and spot, etc., the light therapy assembly can repair the skin, reduce the stinging sensation brought about by the skin damage, and improve the user's sense of experience.

Second, the lamp head is provided separately from the body and is physically and electrically connected through a connection cable, so that when using the beautification instrument, only the lamp head needs to be held, on the one hand, the weight of the lamp head is small, which can reduce the weight, and on the other hand, the volume of the lamp head is small, which can be operated flexibly, thereby improving the user's sense of experience.

Further, the connection cable is detachably connected to at least one of the body and the lamp head, while facilitating overhaul or replacement of the lamp head or the body. The connection cable includes an elastic portion that can be stretched when subjected to an external force, so that when using the beautification instrument, it will not be limited by the length of the adapter cable, which makes it easy to use, and it is naturally retracted to be stowed easily when the external force is removed.

Third, the body and the lamp head are snapped by the connection assembly for fixation, and electrically connected by the plug-in connection terminal set, which enables removal and replacement of the optical hair removal assembly and the body, and the operation is very convenient and simple.

Further, the beautification instrument includes a plurality of lamp heads with different power and different shapes of light outlets, so that the user can select a suitable lamp head according to his or her own usage needs. Therefore, the beautification instrument can be used in a variety of ways to meet various usage needs. It avoids the user from repeatedly purchasing the beautification instrument with different functions for many times, resulting in a waste of resources.

Fourth, since the temperature sense element is provided at the front end of the second housing, and the temperature sense element is protruded forwardly out of the second housing, while facilitating contact with the user's skin, to detect a temperature signal of the user's skin, and control the beautification instrument to stop working when the detected temperature signal exceeds a preset value, thereby preventing the user's skin from being scalded due to the temperature is too high.

Further, since a heat conductive plate is provided at the front end of the temperature sense element, the temperature sense element is in contact with the heat conductive plate, and it can directly contact the user's skin, which can expand the area over which the temperature sense element collects the temperature signal. When the beautification instrument is used, the heat conductive plate is in contact with the user's skin, and the temperature sense element detects the temperature signal of the user's skin by detecting the temperature of the heat conductive plate, and when the temperature signal detected by the temperature sense element exceeds a preset value, the beautification instrument is controlled to stop working, thereby preventing the user's skin from being burned.

It is understood that for those skilled in the art, equivalent substitutions or changes may be made in accordance with the technical solution of the present application and its inventive concept, and all such changes or substitutions shall fall within the scope of the appended claims of the present application.

## Claims

1. A beautification instrument, **characterized by** comprising:
a body, comprising a first housing and a control main board provided inside the first housing;
a lamp head, electrically connected to the control main board, wherein the control main board is configured to to provide control signals and working current for the lamp head, and the lamp head comprises a second housing, and a pulse light source assembly and a light therapy assembly provided within the second housing;
wherein the second housing is provided separately from the first housing, and a light outlet channel is provided inside the second housing, wherein the pulse light source assembly is opposite to the light outlet channel, and pulse light emitted by the pulse light source assembly is ejectable from a front end of the light outlet channel, and light is emitted by the light therapy assembly in a same direction as light emitted by the pulse light source assembly.

2. The beautification instrument according to claim 1, wherein the beautification instrument comprises a connection assembly, the body and the lamp head are connected via the connection assembly.

3. The beautification instrument according to claim 2, wherein the lamp head is provided separately from the body; and
the connection assembly is a connection cable, an end of the connection cable is physically and electrically connected to the body, and another end of the connection cable is physically and electrically connected to the lamp head.

4. The beautification instrument according to claim 3, wherein the connection cable is detachably connected to at least one of the body and the lamp head;
wherein an end of the connection cable is electrically connected to the lamp head and another end of the connection cable is electrically connected to the body, and a control main board of the body is electrically connected to the lamp head via the connection cable.

5. The beautification instrument according to claim 4, wherein two ends of the connection cable are plugged with the body and the lamp head, respectively;
wherein the connection cable comprises an elastic portion, a first connector and a second connector provided at two ends of the elastic portion, wherein the body is provided with a first interface, the lamp head is provided with a second interface, the first connector is pluggable into the first interface, and the second connector is pluggable into the second interface.

6. The beautification instrument according to claim 3, wherein an end of the connection cable is fixed to the body, and an electrical connection of the connection cable and the body is kept, and another end of the connection cable is fixed to the lamp head, and an electrical connection of the connection cable and the lamp head is kept.

7. The beautification instrument according to claim 2, wherein the body is snapped to the lamp head by the connection assembly;
the connection assembly is a snap-fit and a snapping seat for cooperation, the snap-fit is provided at a front end of the body and the snapping seat is provided at a rear end of the lamp head; or
the snap-fit is provided at a rear end of the lamp head and the snapping seat is provided at a front end of the body.

8. The beautification instrument according to claim 7, wherein the snap-fit is protruded at the rear end of the second housing, and the snap-fit comprises a convex extension and a snapping portion, an angle is formed between the snapping portion and the convex extension, and the convex extension is connected to a rear end of the second housing; and
the snap-fit is provided at the front end of the first housing, the snap-fit is provided with a snapping groove with an opening forward, and the snapping portion is snapped in the snapping groove when the lamp head is docked with the body.

9. The beautification instrument according to claim 7, wherein the connection assembly further comprises a limitation member, the limitation member is provided in one of the body and the lamp head, and another one of the body and the lamp head is provided with a limitation groove corresponding to the limitation member, wherein the limitation member is movably accessed into the limitation groove to lock the lamp head and the body.

10. The beautification instrument according to claim 9, wherein the limitation member is provided at the front end of the first housing, wherein the limitation member comprises a pressing portion and a limitation portion, the pressing portion is protruded from one of sides of the first housing, and the limitation portion is protruded from the front end of the first housing; and
the limitation groove is provided at a rear end of the second housing, and the limitation portion is movably accessed into the limitation groove.

11. The beautification instrument according to claim 7, wherein plug-in connection terminal sets adapted to be connected to each other are provided at surfaces where the body is docked with the lamp head.

12. The beautification instrument according to claim 11, wherein the plug-in connection terminal sets comprises a first connection terminal and a second connection terminal, wherein the first connection terminal is provided at the front end of the body, the second connection terminal is provided at the rear end of the lamp head, and the first connection terminal is pluggable into the second connection terminal.

13. The beautification instrument according to claim 12, wherein the connection assembly further comprises a first insertion groove provided at the front end of the body and a second insertion groove provided at the rear end of the lamp head, wherein the first connection terminal is provided in the first insertion groove, the second connection terminal is provided in the second insertion groove, and the first connection terminal is pluaggable with the second connection terminal when the first insertion groove is pluagged in the second insertion groove.

14. The beautification instrument according to claim 1, wherein the light therapy assembly is provided at a front end of the second housing;
the light therapy assembly comprises a light plate with a plurality of light emitting diode (LED) light beads, the light plate is provided with through holes in communication with the light outlet channel; and
the plurality of the LED light beads are electrically connected to the light plate, and provided on the light plate along a circumferential direction of the light plate, and the plurality of light outlets are provided on a front end of the second housing, and each of the plurality of the light outlets is provided with one LED light bead.

15. The beautification instrument according to claim 1, wherein the light therapy assembly is provided at a rear end inside the light outlet channel;
wherein the light therapy assembly comprises a light plate and the plurality of the LED light beads, and the light plate is provided with through holes in communication with the light outlet channel; and
wherein the plurality of the LED light beads are electrically connected to the light plate , provided on the light plate along the circumferential direction of the light plate , and towards an outlet end at the front end of the light outlet channel.

16. The beautification instrument according to claim 1, wherein a temperature sense element is provided at the front end of the second housing, a mounting hole is provided at the front end of the second housing, wherein the temperature sense element is accommodated in the mounting hole and passed through the mounting hole, and is configured to detect a temperature signal from a skin.

17. The beautification instrument according to claim 16, wherein a plurality of the temperature sense elements are provided at the front end of the second housing in a circumferential direction, each of a plurality of mounting holes is provided with one temperature sense element, and the plurality of the temperature sense elements are passed through the plurality of the the mounting holes one-in-one.

18. The beautification instrument according to claim 16, wherein the lamp head further comprises a heat conductive plate provided on the front end of the second housing, and the heat conductive plate is provided in a front end of the temperature sense element, the temperature sense element is in contact with the heat conductive plate; and
the heat conductive plate is provided with an opening hole in communication with the light outlet channel.

19. The beautification instrument according to claim 1, wherein the lamp head further comprises a spotlight frame provided in the second housing, wherein the spotlight frame is extended in a length direction of the beautification instrument, and has openings at front and rear ends of the spotlight frame to form the light outlet channel, and the spotlight frame has an inner diameter that gradually increases from front to back;
wherein the lamp head further comprises a support frame provided around an outer periphery of the spotlight frame.

20. The beautification instrument according to claim 1, wherein the pulse light source assembly comprises a light source, a reflector, and an optical filter, wherein the light source is fixed on an inner side of the reflector, and the light source is a xenon gas tube;
the second housing is also provided with a mounting frame, wherein the mounting frame is located at a rear end of the reflector and fixed to the reflector; and
the optical filter is located at a front end of the reflector and at a rear end of the light outlet channel.
